# EUROPÄISCHE PATENTSCHRIFT

(11) **EP 3 972 623 B1**
(45) Veröffentlichungstag und Bekanntmachung des Hinweises auf die Patenterteilung: **14.10.2026**
(21) Anmeldenummer: 20726480.5
(22) Anmeldetag: 22.05.2020
(51) Int. Cl.: A61K 36/48, A61K 36/47, A61K 36/63, A61K 8/92, A61K 9/00, A61K 31/575, A61K 36/54, A61K 36/738, A61K 47/44, A61Q 19/00, A61Q 19/10

(54) **NATURSTOFF-ZUSAMMENSETZUNG ZUR TOPISCHEN BEHANDLUNG UND PFLEGE DER PSORIATISCHEN HAUT UND ANDERER HAUTERKRANKUNGEN**
TOPICAL COMPOSITION FOR THE TREATMENT AND CARE OF PSORIATIC SKIN
COMPOSITION POUR LE TRAITEMENT TOPIQUE ET LES SOINS DE LA PEAU PSORIATIQUE

(30) Priorität: 23.05.2019 EP 19176178
(43) Veröffentlichungstag der Anmeldung: 30.03.2022
(73) Patentinhaber: Tsakouridou, Despina, 3025 Altona North (AU)
(72) Erfinder: Tsakouridis, Dimitrios, 70439 Stuttgart (DE)
(74) Vertreter: Avon, Philipp Marcel
(86) Internationale Anmeldenummer: PCT/EP2020/064296
(87) Internationale Veröffentlichungsnummer: WO 2020/234460

(56) Entgegenhaltungen:
- WO-A2-2008/065514
- CN-A- 109 771 612
- DE-C1- 4 416 927
- DE-T5- 112009 005 131
- US-B1- 6 383 499

## Beschreibung

Psoriasis ist eine chronisch verlaufende, entzündliche, nicht-infektiöse Hautkrankheit.

Die sich bei gesunden Menschen normalerweise in einem regelmäßigen Zyklus von ca. 26-28 Tagen erneuernde Oberhaut stößt während der Erneuerung fortlaufend gealterte und dabei verhornte Hautzellen ab. Diese Erneuerung ist bei Psoriasispatienten gestört, sodass es zu einer stark beschleunigten Erneuerungsreaktion der Oberhaut kommt, die sich in scharf begrenzten, geröteten, erhobenen Hautbereichen (sogenannten Plaques) äußert, die von einer schuppenden, teilweise silbrig glänzenden Schuppenschicht bedeckt sein können. Am häufigsten sind die Gelenke (z.B. Ellenbogen, Knie), die Kopfhaut und der Genitalbereich betroffen, aber auch die Hautanhangsgebilde wie die Nägel von Fingern oder Zehen können psoriatische Schäden aufweisen. Psoriasis geht häufig mit Begleitkrankheiten wie Arthritis oder Herz-Kreislauferkrankungen einher.

Insgesamt hängt der Verlauf und das Auftreten von sogenannten "Schüben" bei Psoriasispatienten von vielen Faktoren ab. Studien haben gezeigt, dass teilweise genetische Dispositionen beteiligt sind, jedoch auch äußere Bedingungen einen starken Einfluss haben können, so beispielsweise Infektionen, Stress, Ernährung, Wetterverhältnisse oder zusätzlich eingenommene Medikamente. Die pathologische Situation von Psoriasispatienten ist vielschichtig und geht mit einer besonders starken psychischen Belastung einher. Da die Haut als ein äußerlich sichtbares Organ des Körpers von anderen Menschen wahrgenommen wird und somit ein wichtiges Kommunikationsmittel darstellt, führt eine sichtbare Hauterkrankung, wie die Psoriasis, häufig zu einer ungerechtfertigten Zurückweisung und Ausgrenzung. Dies belastet die meisten Psoriasispatienten psychisch. Eine konsequente Behandlung der äußerlich sichtbaren Symptome ist daher grundsätzlich geboten. Neben einer systemischen Behandlung des Patienten, sind verschiedene Licht- oder Elektrotherapiemöglichkeiten bekannt. Vor allem ist jedoch die topische Behandlung von psoriatischer Haut von Bedeutung.

Bekannte Zusammensetzungen zur topischen Behandlung und Pflege der psoriatischen Haut enthalten beispielsweise Kortison, antiseptisch wirksames Dithranol (Anthralin) (so in DE 32 39 203), die Zellproliferation hemmende Vitamin D Derivate, wie Calcipotriol oder Calcitriol (so in WO2008/065514, EP2515865 oder WO2012/127037) oder Salizylsäure und Harnstoff als Keratolytika. Diese Mittel rufen jedoch bei vielen Patienten Unverträglichkeiten hervor und/oder sind aufgrund ihrer Wirkungsweise ausschließlich nach ärztlicher Beratung einzusetzen und auf eine lokale Anwendung der betroffenen Stellen beschränkt.

Oft lindern Produkte mit den o.g. Substanzen die Symptome nur vorübergehend. Psoriasispatienten müssen bei diesen Behandlungen eine genaue Dosierung einhalten und dürfen die Produkte ausschließlich auf die betroffenen Stellen aufbringen. So kann die Haut bei einer Behandlung mit Kortison-haltigen Mitteln dünn und brüchig werden, was gerade für die bereits belasteten Psoriasispatienten von großem Nachteil ist. Dithranol-haltige Produkte können zu Verfärbungen, Hautrötungen und zusätzlichem Juckreiz auf der Haut führen, sodass bei ihnen häufig ein Abwaschen nach einer bestimmten Einwirkzeit notwendig ist. Steinkohleteer-haltige Produkte erhöhen die Empfindlichkeit der Haut für UV-Strahlen und stehen unter Verdacht, Krebs zu erregen.

WO2011/016812A1 offenbart eine Zusammensetzung zur Behandlung von Psoriasis, umfassend a) ein Gemisch aus Ölen, ausgewählt aus Chiaöl, Lebertran, Rizinusöl, Erdnussöl und Olivenöl, b) reines Wachs und Lanolin, c) metallisches Jod, Kampfer und Benzoesäure als Antiseptika, d) Chlorophyll und/oder Kräuterextrakte und e) einen pharmazeutisch verträglichen Trägerstoff. Diese Zusammensetzung enthält synthetische Bestandteile und ist folglich keine Zusammensetzung aus Naturstoffen, die ausschließlich natürlich vorkommende pflanzliche oder tierische Stoffe enthält, die unverarbeitet sind oder lediglich durch mechanische oder wasserbasierte Extraktionsmethoden gewonnen werden. Metallisches Jod ist ein bei Raumtemperatur grauschwarzer, metallisch glänzender Feststoff, der zu Hautverfärbungen führt und zur Allergisierung führen kann. Kampfer kann ebenfalls zu Verfärbungen, Hautrötungen und zusätzlichem Juckreiz auf der Haut führen, sodass diese Zusammensetzung mit Nebenwirkungen verbunden ist.

Psoriasispatienten stehen derartigen Wirkstoffen daher häufig skeptisch gegenüber und suchen nach Alternativen, die sich in der Dauerbehandlung einfacher einsetzen lassen und keine der oben genannten Nebenwirkungen aufweisen. Sie sind besonders aufmerksam und kritisch gegenüber den Inhaltsstoffen von Produkten zur Pflege und Behandlung psoriatischer Haut. Insbesondere Patienten aus Risikogruppen, für die eine besondere Vorsicht bei Medikationen gilt, wie beispielsweise Kinder oder Schwangere, benötigen alternative Behandlungsmöglichkeiten.

Die Fachwelt ist daher bereits seit einiger Zeit dazu übergegangen, Produkte zur Pflege und Behandlung psoriatischer Haut auf Basis von Naturstoffen herzustellen. Naturstoffe umfassen dabei alle chemischen Verbindungen, die von Organismen gebildet werden, um eine biologische Funktion zu erfüllen. Naturstoffe können aufgrund ihrer häufig hohen strukturellen Diversität nur schwer vollsynthetisch hergestellt werden, sodass sie meist als Extrakt aus pflanzlichen oder tierischen Bestandteilen gewonnen werden.

Die in Naturstoffen enthaltenen Biomoleküle umfassen biologisch aktive Substanzen, die gerade in der Pharmazeutik eine wichtige Rolle spielen.

So offenbart beispielsweise EP 3 013 423 B1 eine Zusammensetzung auf Basis von Ölen und Wachsen, mit einem öligen Auszug aus Nesseln (*Oleum coctus Urticae*). Das Produkt wird unter anderem auch zur Behandlung der Haut bei Psoriasis eingesetzt.

EP 2 489 358 B1 führt den ebenfalls durch Ölextraktion gewonnenen Naturstoff Indirubin aus *Indigo naturalis* zur Behandlung der Psoriasis an. Bevorzugt werden auch hier pflanzliche Öle verwendet.

Auch EP 1 267 900 B1 nennt einen Naturstoff, Betulin, der im Wesentlichen Triterpene umfasst, welcher in Form einer Emulsion verarbeitet wird, um auf die psoriatische Haut aufgetragen zu werden.

Auch wenn die vorgenannten, auf Naturstoffen basierenden Produkte eine höhere Akzeptanz bei den Patienten haben als herkömmliche Medikamente zur Behandlung der psoriatischen Haut, sind jedoch weiterhin Nachteile in der Herstellung und der Anwendung zu verzeichnen.

Die genannten Naturstoffe werden jeweils in aufwändigen Verfahren extrahiert und weiter zu bestimmten Darreichungsformen, meist als Hautöl oder Emulsion, verarbeitet. Teilweise, so beispielsweise für den Wirkstoff Betulin, müssen Lösungsmittel verwendet werden. Die Herstellungsverfahren für die Produkte sind daher aufwändig und kostenintensiv.

Auch wenn es sich um Naturstoffe handelt, können unerwünschte Nebenwirkungen nicht immer ausgeschlossen werden. So sind Unverträglichkeiten oder Überreaktionen auf Nesseln bekannt. Auch wenn bereits Verfahren zur Herstellung von Indirubinhaltigen Produkten bekannt sind, die die Hautfärbung durch Indirubin reduzieren, färbt die Substanz weiterhin die Haut. Patienten, die bereits schon aufgrund der sichtbaren Hautkrankheit psychisch belastet sind, schrecken deshalb auch vor dieser Art von Produkten zurück.

Der vorliegenden Erfindung liegt daher die Aufgabe zugrunde, eine Zusammensetzung zur Behandlung und Pflege der psoriatisch erkrankten Haut aus Naturstoffen bereitzustellen, die auch als Naturstoff-Zusammensetzung bezeichnet wird, welche für möglichst alle betroffenen Patienten risikoarm auf der Haut angewendet werden kann, aufgrund fehlender Nebenwirkungen gut verträglich ist, zur vollständigen Abheilung psoriatischer Haut führt und dabei trotzdem mit geringem Kosten- und Produktionsaufwand herzustellen ist.

Die Erfindungsaufgabe wird gelöst durch eine Zusammensetzung aus Naturstoffen (oder Naturstoff-Zusammensetzung) gemäß Anspruch 1, welche aus Erdnussöl (*Arachis hypogaea*), Olivenöl *(Oleum olivarum),* Rizinusöl (*Ricinus communis*) und Lanolin sowie gegebenenfalls einem oder mehreren Vertreter(n), ausgewählt aus der Gruppe bestehend aus Avocadoöl (*Persea gratissima*), Rosenöl (*Rosa damascena*), Rosenhydrolat und Phytosterol besteht.

Erfindungsgemäß besteht die Zusammensetzung ausschließlich aus natürlich vorkommenden pflanzlichen oder tierischen Stoffen, die unverarbeitet sind oder lediglich durch mechanische oder wasserbasierte Extraktionsmethoden (Flotation, Extraktion mit Wasser, Dampfdestillation usw.) gewonnen werden. Die erfindungsgemäß in der Zusammensetzung enthaltene Mischung aus wenigstens Erdnussöl, Olivenöl, Rizinusöl und Lanolin, sowie wahlweise Rosenöl und/oder Rosenhydrolat und/oder Avocadoöl und/oder Phytosterol besteht demnach aus ausschließlich auf die oben genannte Weise gewonnenen Substanzen. Die erfindungsgemäße Zusammensetzung ist demnach vollständig natürlichen Ursprungs, d.h. sie enthält keine Bestandteile, die synthetischen oder künstlichen Ursprungs sind.

Die erfindungsgemäße Zusammensetzung besteht überwiegend aus pflanzlichen Ölen, die in ihrer Mischung mit Lanolin einen höchst positiven Effekt auf die psoriatische Haut oder Ekzeme, frische Narben, Schwangerschaftsstreifen, trockene Haut, Hautauschläge und durch Juckreiz oder Sonnenbrand geschädigte Haut zeigen. Die Verhornungen lösen sich und die entzündete, gerötete Haut beruhigt sich. Die erfindungsgemäße Mischung ist besonders verträglich, zeigt keinerlei Nebenwirkungen und kann daher bedenkenlos von allen Patientengruppen äußerlich angewendet werden. Bei regelmäßiger, d.h. z.B. zwei- oder dreimal täglicher, Anwendung verbessert sich das Krankheitsbild wesentlich. Insbesondere gehen die Entzündungen der Haut deutlich zurück. Die erfindungsgemäße Zusammensetzung kann bei regelmäßiger Anwendung außerdem zur vollständigen Abheilung psoriatischer Haut führen. Für eine vollständige Abheilung wird eine zweibis dreimal tägliche Verabreichung über einen Zeitraum von mindestens ein bis drei Monaten empfohlen.

Auch Psoriasispatienten oder an Ekzemen, an frischen Narben, an Schwangerschaftsstreifen, an trockener Haut, an Juckreiz, an Hautauschlägen und an Sonnenbrand leidende Patienten, die besonderen Risikogruppen angehören, so beispielsweise Kinder und Schwangere, können die erfindungsgemäß Zusammensetzung ohne Gefahr anwenden.

Die erfindungsgemäße Zusammensetzung hat den Vorteil, dass sie die Haut nicht verfärbt, wie es bei anderen in der Psoriasisbehandlung bekannten Mitteln, beispielsweise Indigohaltigen oder Jod-haltigen Mitteln (siehe WO2011/016812A1), der Fall ist.

Die Verwendung einer geringen Zahl an Inhaltsstoffen bei der erfindungsgemäßen Zusammensetzung hat den Vorteil, dass der Patient sich einen raschen Überblick über die Inhaltsstoffe verschaffen kann. Auch das Allergierisiko wird so stark begrenzt.

Letztendlich ist die Herstellung der erfindungsgemäßen Zusammensetzung mit einer einfachen Einrichtung möglich. Zudem sind die Herstellungskosten gering.

Bevorzugt besteht die erfindungsgemäße Zusammensetzung aus Erdnussöl, Olivenöl, Rizinusöl und Lanolin. Bevorzugt besteht die erfindungsgemäße Zusammensetzung aus Erdnussöl, Olivenöl, Rizinusöl und Lanolin in den Konzentrationen Erdnussöl 50-60 vol.%, Olivenöl 25-35 vol.%, Rizinusöl 8-14 vol.%, sowie Lanolin 1-4 vol.%. Die tatsächlich gewählten Konzentrationen ergeben dabei in der Summe 100 vol.% (Volumenprozent).

"Erdnussöl" ist ein aus den Samen der Erdnuss (*Arachis hypogaea*) gepresstes Pflanzenöl. Aufgrund des Erdnussöls ist die erfindungsgemäße Zusammensetzung nicht für Erdnuss-Allergiker geeignet.

"Olivenöl", auch Baumöl oder fachsprachlich *Oleum olivarum* genannt, ist ein aus dem Fruchtfleisch und aus dem Kern von Oliven, den Früchten des Ölbaums (*Olea europaea*), gewonnenes Pflanzenöl.

"Rizinusöl" ist ein aus den Samen des Wunderbaumes (*Ricinus comunis*) und anderen Mitgliedern der Familie *Euphorbisceae* gewonnenes Pflanzenöl, wobei das Episperma aus den Samen entfernt wurde.

"Lanolin", das auch als Wollwachs bezeichnet wird, ist das Sekret aus den Talgdrüsen von Schafen, welches aus Schafwolle gewonnen wird.

Ebenfalls kann die erfindungsgemäße Zusammensetzung aus Erdnussöl, Olivenöl, Rizinusöl, Lanolin und Rosenöl bestehen. Bevorzugt kann die erfindungsgemäße Zusammensetzung aus Erdnussöl, Olivenöl, Rizinusöl, Lanolin und Rosenöl jeweils in den folgenden Konzentrationen bestehen: Erdnussöl 50-60 vol.%, Olivenöl 25-35 vol.%, Rizinusöl 8-14 vol.%, Lanolin 1-3 vol.%, sowie Rosenöl 0,001-0,020 vol.%, wobei die gewählten Konzentrationen in der Summe 100 vol.% ergeben.

"Rosenöl" wird mittels Wasserdampf-Destillation aus den Blütenblättern von Rosen (meist *Rosa centifolia* oder *Rosa damascena*) gewonnen. Das Rosenöl wirkt zusammen mit den anderen Naturstoffen der erfindungsgemäßen Zusammensetzung besonders positiv auf die betroffenen Hautbereiche ein.

Gemäß einer weiteren Ausführungsform kann die erfindungsgemäße Zusammensetzung auch aus Erdnussöl, Olivenöl, Rizinusöl, Lanolin und Rosenhydrolat bestehen, wobei die Konzentrationen bevorzugt wie folgt gewählt sind: Erdnussöl 50-60 vol.%, Olivenöl 25-33 vol.%, Rizinusöl 8-14 vol.%, Lanolin 1-4 vol.%, sowie Rosenhydrolat 1-3 vol.% und in der Summe 100 vol.% ergeben.

"Rosenhydrolat" bzw. Rosenwasser entsteht als Nebenprodukt bei der Destillation von Rosenöl aus Rosenblüten. Es enthält in besonders hohem Anteil 2-Phenylethanol. Rosenhydrolat wirkt auf die Haut entzündungshemmend und gleichzeitig feuchtigkeitsspendend.

Eine weitere mögliche Ausführungsform der erfindungsgemäßen Zusammensetzung besteht aus Erdnussöl, Olivenöl, Rizinusöl, Avocadoöl, Lanolin und Phytosterol, wobei die folgenden Konzentrationen bevorzugt sind: Erdnussöl 50-60 vol.%, Rizinusöl 25-35 vol.%, Olivenöl 5-12 vol.%, Avocadoöl 2-8 vol.%, Lanolin 1-4 vol.%, sowie Phytosterol 0,2-0,5 vol.%, wobei die Konzentrationen der einzelnen Substanzen in der Summe 100 vol.% ergeben.

Avocadoöl ist ein Pflanzenöl, das aus den Früchten des Avocadobaums (Persea americana) gewonnen wird. Es ist wie das Olivenöl ein Fruchtfleischöl.

Phytosterole (oder auch Phytosterine) sind sekundäre Pflanzenstoffe, die mindestens 70% beta-Sitosterol umfassen. Erfindungsgemäß wird bevorzugt pflanzliches Phytosterol eingesetzt, welches bevorzugt als weißer Feststoff vorliegt. Bevorzugt wird Pinus-Phytosterol (Pinus SSP), wie z.B. das Phytosterol mit der CAS-Nr. 949109-75-5 (PHYTOPIN^{®}), eingesetzt. Dieses Phytosterol umfasst 70-80% beta-Sitosterol, maximal 15% Sitostanol, maximal 6-10% Campesterol 6-10, maximal 2% Campestanol und maximal 3% andere Sterole.

Die erfindungsgemäße Zusammensetzung kann darüber hinaus auch aus Erdnussöl, Olivenöl, Rizinusöl, Avocadoöl, Lanolin, Rosenöl und Phytosterol bestehen, wobei die folgenden Konzentrationen bevorzugt sind: Erdnussöl 50-60 vol.%, Rizinusöl 25-35 vol.%, Olivenöl 3-7 vol.%, Avocadoöl 4-8 vol.%, Lanolin 2-4 vol.%, Rosenöl 0,01-0,02 vol.%, sowie Phytosterol 0,2-0,5 vol.%, wobei die gewählten Konzentrationen in der Summe 100 vol.% ergeben.

Erfindungsgemäß können außerdem das Rizinusöl und/oder das Olivenöl in ozonisierter Form vorliegen. Die mit Sauerstoff angereicherten Öle bewirken, dass die Haut verstärkt mit Sauerstoff versorgt wird, was die Abheilung der betroffenen Hautstellen beschleunigt.

Die erfindungsgemäße Zusammensetzung kann erfindungsgemäß auch als alleinig zur Behandlung der psoriatischen Haut wirksamer Bestandteil Inhaltsstoff eines Körperpflegemittels sein, insbesondere einer Lotion, einer Creme, einer Salbe, einer Waschlotion oder eines Shampoos.

Des Weiteren ist es im Rahmen der Erfindung vorgesehen, die Zusammensetzung auf einem Trägermaterial darzureichen, welches zur Auflage auf der zu behandelnden psoriatischen Haut vorgesehen ist und wobei das Trägermaterial eine bestimmte Menge der erfindungsgemäßen Zusammensetzung zur Einwirkung auf die zu behandelnde Haut enthält. Die erfindungsgemäße Zusammensetzung kann auf diese Weise über einen längeren Zeitraum, d.h. über mehrere Stunden, aus dem Arzneipflaster heraus auf die Haut einwirken, was den Behandlungserfolg weiter verbessert. Darüber hinaus wird vorteilhafterweise die Kleidung des Patienten geschont, da ein Kontakt mit der Zusammensetzung vermieden werden kann.

Im Folgenden werden fünf mögliche Ausführungsformen der Erfindung genannt:

**Tab. 1: Ausführungsform 1**

| **Ausführungsform** I | |
|---|---|
| **Inhaltsstoffe** | **Konzentration vol.%** |
| Erdnussöl (*Arachis Hypogaea*) | 55,00 |
| Rizinusöl (*Ricinus Communis*) | 30,00 |
| Olivenöl (*Oleum olivarum*) | 6,50 |
| Avocadoöl (*Persea Gratissima*) | 6,00 |
| Lanolin | 2,25 |
| Phytosterol (pflanzlich) | 0,25 |

**Tab. 2: Ausführungsform 2**

| **Ausführungsform II** | |
|---|---|
| **Inhaltsstoffe** | **Konzentration vol.%** |
| Erdnussöl (*Arachis Hypogaea*) | 55,98 |
| Rizinusöl (*Ricinus Communis*) | 30,00 |
| Olivenöl (*Oleum olivarum*) | 5,00 |
| *Avocadoöl* (*Persea Gratissima*) | 5,75 |
| Lanolin | 3,00 |
| Rosenöl (*Rosa Damascena*) | 0,02 |
| Phytosterol (pflanzlich) | 0,25 |

**Tab. 3: Ausführungsform 3**

| **Ausführungsform III** | |
|---|---|
| **Inhaltsstoffe** | **Konzentration vol.%** |
| Erdnussöl (*Arachis Hypogaea*) | 55,00 |
| ozonisiertes Rizinusöl (*Ricinus Communis)* | 30,00 |
| ozonisiertes Olivenöl (*Olea Europaea*) | 10,00 |
| Lanolin | 2,40 |
| Avocadoöl (*Persea Gratissima)* | 2,35 |
| Phytosterol (pflanzlich) | 0,25 |

**Tab. 4: Ausführungsform 4**

| **Ausführungsform IV** | |
|---|---|
| **Inhaltsstoffe** | **Konzentration vol.%** |
| Erdnussöl *(Arachis Hypogaea)* | 55,98 |
| Olivenöl (*Oleum olivarum*) | 30,00 |
| Rizinusöl (*Ricinus Communis*) | 12,00 |
| Lanolin | 2,00 |
| Rosenöl (*Rosa Damascena*) | 0,02 |

**Tab. 5: Ausführungsform 5**

| **Ausführungsform V** | |
|---|---|
| **Inhaltsstoffe** | **Konzentration vol.%** |
| Erdnussöl (*Arachis Hypogaea*) | 55,0 |
| Olivenöl (*Oleum olivarum*) | 28,0 |
| Rizinusöl (*Ricinus Communis*) | 10,5 |
| Lanolin | 3,0 |
| Rosenwasser | 1,5 |

**Tab. 6: Ausführungsform 6**

| **Ausführungsform VI** | |
|---|---|
| **Inhaltsstoffe** | **Konzentration vol.%** |
| Erdnussöl (*Arachis Hypogaea*) | 54,98 |
| ozonisiertes Rizinusöl (*Ricinus Communis*) | 30,00 |
| ozonisiertes Olivenöl (*Olea Europaea*) | 10,00 |
| Lanolin | 2,40 |
| Avocadoöl (*Persea Gratissima*) | 2,35 |
| Phytosterol (pflanzlich) | 0,25 |
| Rosenöl (*Rosa damascena*) | 0,02 |

Die erfindungsgemäße Naturstoff-Zusammensetzung kann 20 bis 24 Monate bei Raumtemperatur gelagert werden. Folglich ist die erfindungsgemäße Naturstoff-Zusammensetzung 20 bis 24 Monate bei Raumtemperatur stabil.

Die erfindungsgemäße Naturstoff-Zusammensetzung kann bevorzugt in Form eines Öls, z.B. eines blassgelben Öls, vorliegen. Dies hat den Vorteil, dass sie leicht in die zu behandelnde Haut eindringt und bevorzugt zu keiner Verfärbung der Haut führt. Alternativ kann die erfindungsgemäße Naturstoff-Zusammensetzung auch in Form einer Creme, eines Gels, einer Lotion oder einer Paste vorliegen. Besonders bevorzugt liegt sie in Form eines Öls vor.

Die oben aufgeführten Zusammensetzungen können vorteilhafterweise mit geringem Aufwand angefertigt werden. Die einzelnen Öle werden nacheinander zusammengemischt und bei 56-58°C erwärmt. Sodann werden Lanolin und gegebenenfalls Phytosterol zugegeben und gelöst. Alternativ wird das Erdnussöl gegebenenfalls mit dem Phytosterol zusammengemischt und auf 50 bis maximal 75°C erwärmt. Im Anschluss werden Olivenöl, Rizinusöl, und gegebenenfalls ein oder mehrere Vertreter, ausgewählt aus der Gruppe bestehend aus Avocadoöl, Rosenöl, und Rosenhydrolat, zusammen mit Lanolin hinzugefügt und die erhaltene Mischung abgekühlt.

Die Wirkung der erfindungsgemäßen Zusammensetzungen in den oben genannten Ausführungsformen wurde bereits bei mehreren Patienten erfolgreich getestet. Die Probanden waren alle von Psoriasis Typ I betroffen. Innerhalb von 8 bis 10 Wochen konsequenter Behandlung mit der erfindungsgemäßen Zusammensetzung waren bei allen Patienten die Plaques in einer bevorzugten Ausführungsform deutlich zurückgegangen, teilweise auch vollständig abgeheilt. Eine "konsequente Behandlung" umfasst erfindungsgemäß, dass der Patient die erfindungsgemäße Zusammensetzung mindestens zweimal täglich (z.B. morgens und abends) in einer dünnen Schicht (d.h. ein paar Tropfen) auf die betroffene Stelle aufträgt, einreibt und wartet bis sie vollständig einzieht, was etwa 15 bis 20 min dauern kann. Ebenso kann die erfindungsgemäße Zusammensetzung dreimal täglich (z.B. morgens, mittags und abends) in einer dünnen Schicht auf die betroffene Stelle aufgetragen werden. Diese Anwendung kann insbesondere Juckreiz sofort lindern.

Untenstehend wird anhand von Beispielen die Wirkung der erfindungsgemäßen Zusammensetzung erläutert.

### Beispiel 1:

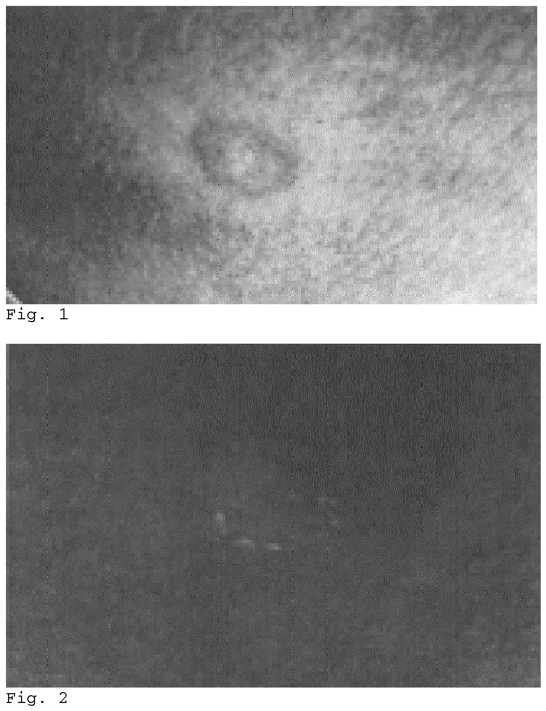

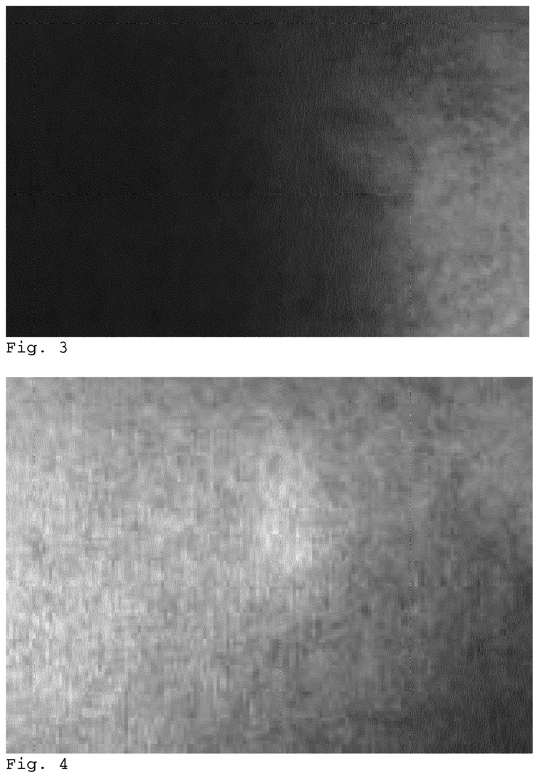

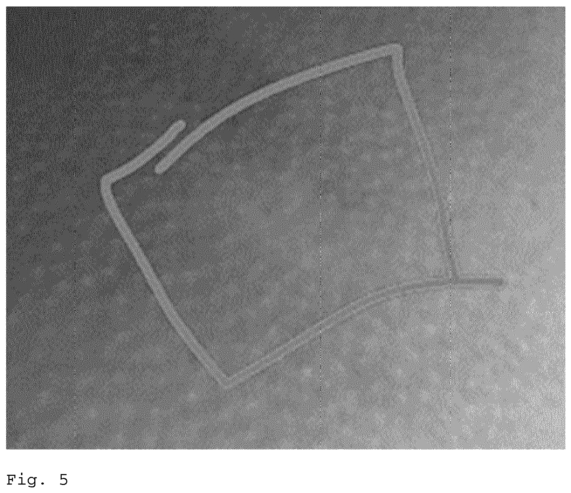

Die Patientin, 19 Jahre, ist von Psoriasis Typ I an Arm und Oberschenkel betroffen, sowie von Psoriasis capitis an der Kopfhaut. Die Plaques an Oberschenkel und Arm wurden mit der erfindungsgemäßen Zusammensetzung (Ausführungsform 4) behandelt. Die Testbehandlung begann am 15. Januar 2018 und endete am 29. März 2018. Die Zusammensetzung wurde morgens und abends auf die betroffenen Stellen aufgebracht. Die Stellen wurden mit einem Pflaster abgedeckt, um die Einwirkung der Zusammensetzung auf die Haut auch unter der Kleidung zu ermöglichen. Die Figuren 1 - 5 zeigen den Verlauf der Heilung der betroffenen Hautstelle am Oberschenkel.

### Dabei zeigt

- Figur 1: die behandelte Hautstelle am Tag 1 der Behandlung,
- Figur 2: die behandelte Hautstelle am Tag 30 der Behandlung,
- Figur 3: die behandelte Hautstelle am Tag 62 der Behandlung,
- Figur 4: die behandelte Hautstelle am Tag 72 der Behandlung, und
- Figur 5: die behandelte Hautstelle am Tag 75 der Behandlung.

### Beispiel 2:

Es wurde eine monozentrische, klinisch-dermatologische Anwendungsstudie mit der erfindungsgemäßen Zusammensetzung gemäß Ausführungsform II und mit 20 Probanden sowie zweimal täglicher Anwendung an den durch Psoriasis hervorgerufenen, akuten Ekzemen über einen Zeitraum von 8 Wochen durchgeführt.

Primäre Studienziele waren hierbei die Prüfung der Hautverträglichkeit (vom Zeitpunkt des Studienbeginns bis zum Studienende und 30 Tage darüber hinaus werden alle Hautreaktionen und mögliche sonstige Nebenwirkungen im Reaktionsbogen erfasst). Sekundäre Studienziele waren hierbei die Prüfung der Wirksamkeit über dermatologisches Assessment (Messzeitpunkte: T_{4 Wochen} und T_{8 Wochen}).

Die Einschlusskriterien waren: 18 Jahre und älter, weibliche und männliche hautgesunde Probanden, Hauttyp: beliebig, Probandinnen und Probanden mit akutem Ekzem bei Psoriasis (nicht-ärztlich behandlungsbedürftig), schriftliche Einverständniserklärung der Probanden bzw. Erziehungsberechtigten liegt vor.

Die Ausschlusskriterien waren: schwere oder chronische Hautentzündungen, schwere innere oder chronische Erkrankungen, Einnahme von Medikamenten, die die Hautreaktion beeinträchtigen können (Glucocorticoide, Antiallergika, topische Immunmodulatoren, etc.), Applikation von wirkstoffhaltigen Präparaten und Pflegemitteln 7-10 Tage vor Testbeginn, schwere Allergien oder jemals aufgetretene schwerwiegende Nebenwirkung durch kosmetische Präparate, Sonnenbäder oder Solariumbesuche während der Studie, bekanntes Krebsleiden, und Schwangerschaft/Stillzeit.

### Dermatologische Untersuchungsergebnisse:

Die Untersuchungen wurden nach klinisch-dermatologischen Beurteilungskriterien durchgeführt. 20 Testpersonen zeigten vor, während und nach der Anwendungsstudie eine gesunde Haut im Bereich des Testareals. Pathologische Hautveränderungen waren in keiner Form festzustellen. Testunterbrechungen oder gar hautfachärztliche Behandlungen wurden in keinem Fall durchgeführt. Die erfindungsgemäße Zusammensetzung wurde von diesen Probanden sehr gut vertragen und führte zu keinerlei dermatologisch relevanten Hautveränderungen.

Eine Probandin gab nach 14 Tagen Anwendung ein schmerzhaftes Brennen an beiden Ellenbogen nach dem Eincremen der von Schuppenflechte betroffenen Haut an. Bei der Untersuchung war das Hautareal auch etwas gereizt und leicht gerötet. Die Haut am akuten Ekzem der Psoriasis war bei der Untersuchung intakt. Die Probandin hatte keine Nussallergie. Die Probandin brach die Studie ab. Die Symptome am Ekzem der Psoriasis klangen nach circa vier Tagen vollständig ab, eine ärztliche Behandlung war nicht erforderlich.

### Dermatologisches Assessment:

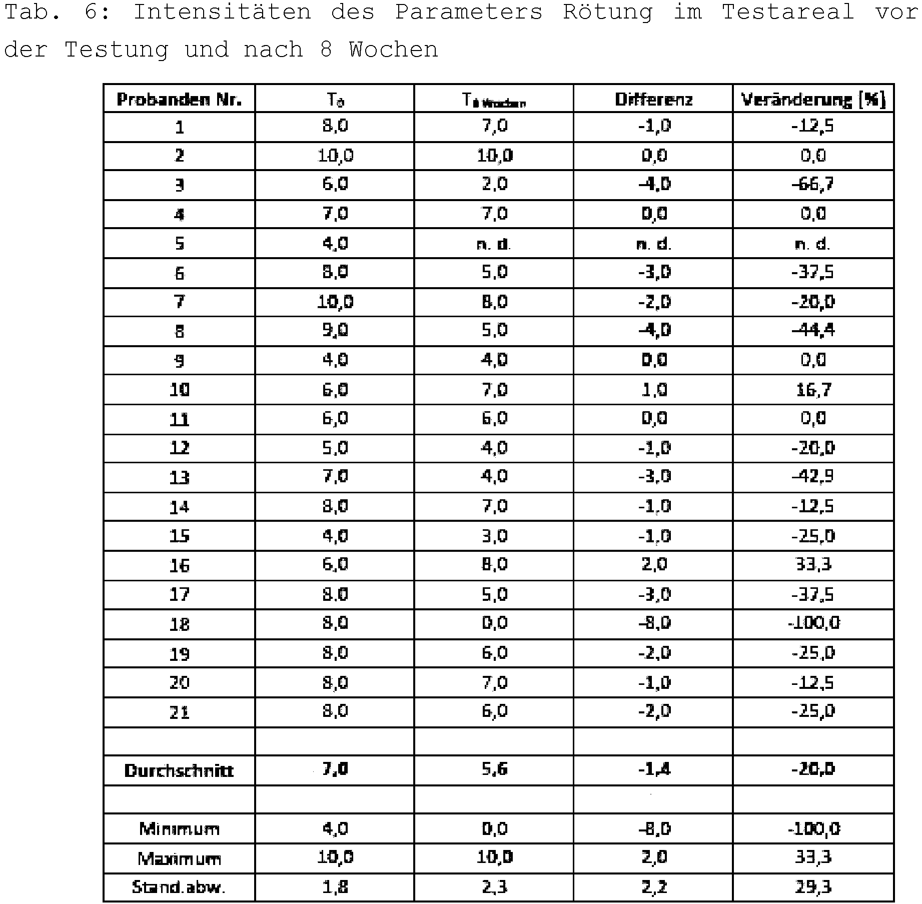

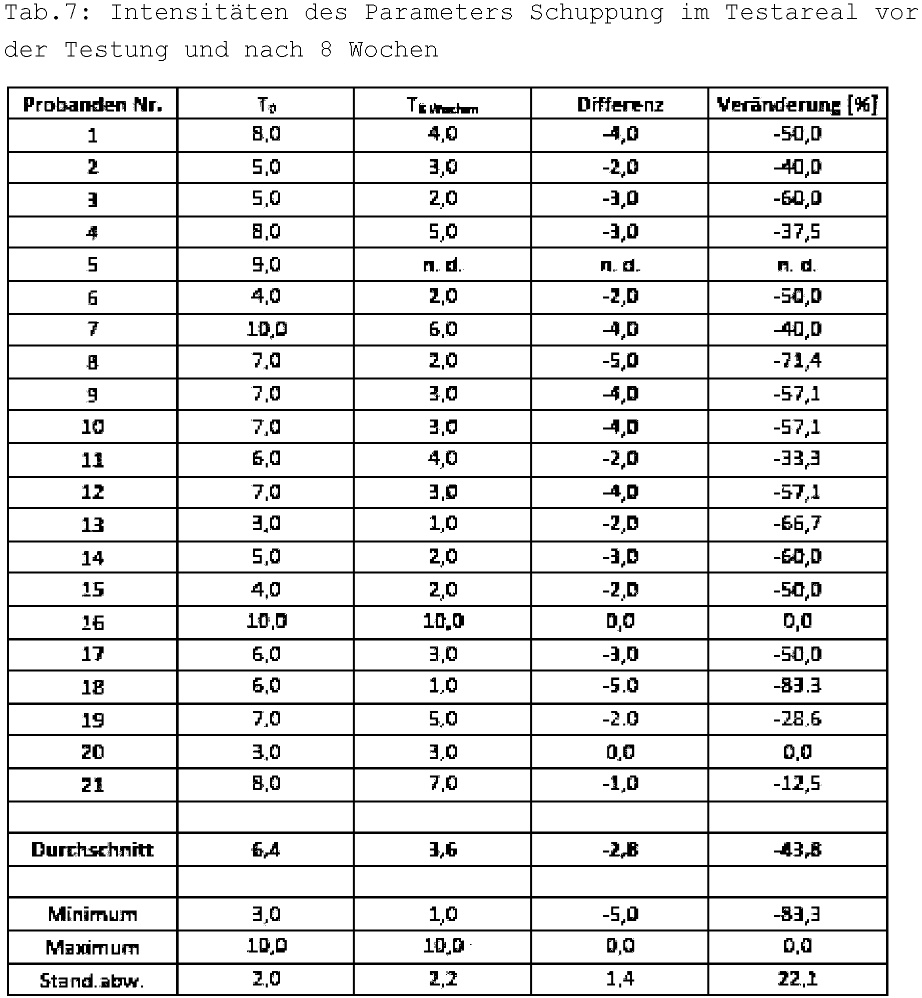

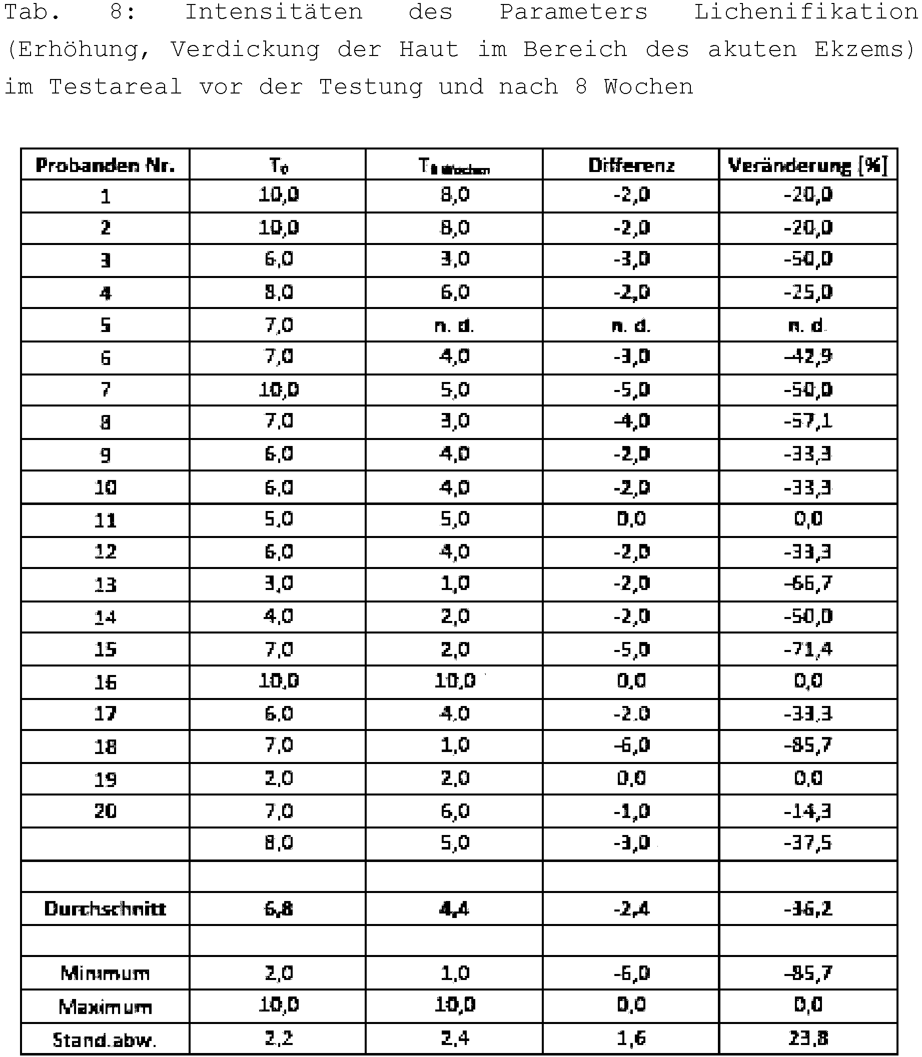

### Beurteilung der Studienergebnisse:

### Hautverträglichkeit:

Die erfindungsgemäße Zusammensetzung wurde über einen Zeitraum von acht Wochen von 20 Probanden zweimal täglich vor allem an den durch die Psoriasis hervorgerufen akuten Ekzeme angewendet. Es kam aus klinisch-dermatologischer Sicht zu keinerlei relevanten Hautreaktionen im Testareal, die erfindungsgemäße Zusammensetzung wurde sehr gut vertragen. Es wurden weder Intoleranzreaktionen im Sinne einer Irritation noch allergische Reaktionen (Kontaktdermatitis) festgestellt. Eine Probandin gab nach 14 Tagen Anwendung ein schmerzhaftes Brennen an beiden Ellenbogen nach dem Eincremen der von Schuppenflechte betroffenen Haut an. Bei der Untersuchung war das Hautareal auch etwas gereizt und leicht gerötet. Die Probandin hat keine Nussallergie. Die Haut am akuten Ekzem der Psoriasis war bei der Untersuchung intakt. Die Symptome am Ekzem der Psoriasis klangen nach circa vier Tagen vollständig ab, eine ärztliche Behandlung war nicht erforderlich.

### Wirksamkeit:

Die Wirksamkeit der erfindungsgemäßen Zusammensetzung in Bezug auf die Symptome einer Psoriasis Rötung, Schuppung und Lichenifikation (Erhöhung der Haut im Bereich des akuten Ekzems) wurde mit Hilfe eines dermatologischen Assessments bestimmt. Es konnte eine Verringerung der Rötung von -20,0 %, der Schuppung von -43,8 % und der Lichenifikation (Erhöhung der Haut im Bereich des akuten Ekzems) von -36,2 % im Testareal gezeigt werden.

## Patentansprüche

1. Zusammensetzung aus Naturstoffen, bestehend aus Erdnussöl, Olivenöl, Rizinusöl und Lanolin sowie gegebenenfalls einem oder mehreren Vertreter(n), ausgewählt aus der Gruppe bestehend aus Avocadoöl, Rosenöl, Rosenhydrolat und Phytosterol.

2. Zusammensetzung gemäß Anspruch 1, **dadurch gekennzeichnet, dass** sie
(i) aus Erdnussöl, Olivenöl, Rizinusöl und Lanolin besteht, wobei sie Erdnussöl, Olivenöl, Rizinusöl und Lanolin in den folgenden Konzentrationen enthält:
- Erdnussöl 50-60 vol.%,
- Olivenöl 25-35 vol.%,
- Rizinusöl 8-14 vol.%, sowie
- Lanolin 1-4 vol.%,
wobei die gewählten Konzentrationen in der Summe 100 vol.% ergeben; oder
(ii) aus Erdnussöl, Olivenöl, Rizinusöl, Lanolin und Rosenöl besteht, wobei sie Erdnussöl, Olivenöl, Rizinusöl, Lanolin und Rosenöl in den folgenden Konzentrationen enthält:
- Erdnussöl 50-60 vol.%,
- Olivenöl 25-35 vol.%,
- Rizinusöl 8-14 vol.%,
- Lanolin 1-3 vol.%, sowie
- Rosenöl 0,001-0,020 vol.%,
wobei die gewählten Konzentrationen in der Summe 100 vol.% ergeben; oder
(iii) aus Erdnussöl, Olivenöl, Rizinusöl, Lanolin und Rosenhydrolat besteht, wobei sie Erdnussöl, Olivenöl, Rizinusöl, Lanolin und Rosenhydrolat in den folgenden Konzentrationen enthält:
- Erdnussöl 50-60 vol.%,
- Olivenöl 25-33 vol.%,
- Rizinusöl 8-14 vol.%,
- Lanolin 1-4 vol.%, sowie
- Rosenhydrolat 1-3 vol.%,
wobei die gewählten Konzentrationen in der Summe 100 vol.% ergeben; oder
(iv) aus Erdnussöl, Olivenöl, Rizinusöl, Avocadoöl, Lanolin und Phytosterol besteht, wobei sie Erdnussöl, Olivenöl, Rizinusöl, Avocadoöl, Lanolin und Phytosterol in den folgenden Konzentrationen enthält:
- Erdnussöl 50-60 vol.%,
- Rizinusöl 25-35 vol.%,
- Olivenöl 5-12 vol.%,
- Avocadoöl 2-8 vol.%,
- Lanolin 1-4 vol.%, sowie
- Phytosterol 0,2-0,5 vol.%,
wobei die gewählten Konzentrationen in der Summe 100 vol.% ergeben; oder
(v) aus Erdnussöl, Olivenöl, Rizinusöl, Avocadoöl, Lanolin, Rosenöl und Phytosterol besteht, wobei sie Erdnussöl, Olivenöl, Rizinusöl, Avocadoöl, Lanolin, Rosenöl und Phytosterol in den folgenden Konzentrationen enthält:
- Erdnussöl 50-60 vol.%,
- Rizinusöl 25-35 vol.%,
- Olivenöl 3-7 vol.%,
- Avocadoöl 4-8 vol.%,
- Lanolin 2-4 vol.%,
- Rosenöl 0,001-0,020 vol.%, sowie
- Phytosterol 0,2-0,5 vol.%,
wobei die gewählten Konzentrationen in der Summe 100 vol.% ergeben.

3. Zusammensetzung gemäß einem der vorangehenden Ansprüche, **dadurch gekennzeichnet, dass** sie aus folgenden Komponenten besteht:
| | |
|---|---|
| Erdnussöl | 55,98 vol.% |
| Rizinusöl | 30,00 vol.% |
| Olivenöl | 5,00 vol.% |
| Avocadoöl | 5,75 vol.% |
| Lanolin | 3,00 vol.% |
| Rosenöl | 0,02 vol.% |
| Phytosterol (pflanzlich) | 0,25 vol.% |

4. Zusammensetzung gemäß einem der vorangehenden Ansprüche, **dadurch gekennzeichnet, dass** sie ausschließlich natürlich vorkommende pflanzliche oder tierische Stoffe enthält, die unverarbeitet sind oder lediglich durch mechanische oder auf Wasser basierenden Extraktionsmethoden gewonnen werden.

5. Zusammensetzung gemäß einem der vorangehenden Ansprüche, **dadurch gekennzeichnet, dass** das Rizinusöl und/oder das Olivenöl in ozonisierter Form vorliegen.

6. Zusammensetzung gemäß einem der vorangehenden Ansprüche, **dadurch gekennzeichnet, dass** sie in Form eines Öls, einer Creme, eines Gels, einer Lotion oder einer Paste vorliegt, wobei sie bevorzugt in Form eines Öls vorliegt.

7. Zusammensetzung gemäß einem der vorangehenden Ansprüche zur topischen Behandlung und Pflege der psoriatischen Haut.

8. Zusammensetzung nach Anspruch 7, wobei die Zusammensetzung zweimal oder dreimal täglich auf die zu behandelnde Haut aufgetragen wird.

9. Verfahren zur Herstellung der Zusammensetzung gemäß einem der Ansprüche 1 bis 7, umfassend die folgenden Schritte:
(i) Mischen von Erdnussöl und gegebenenfalls Phytosterol,
(ii) Erwärmen der erhaltenen Mischung auf 50 bis 75°C,
(iii) Zugabe von Olivenöl, Rizinusöl, und gegebenenfalls einem oder mehrerer Vertreter, ausgewählt aus der Gruppe bestehend aus Avocadoöl, Rosenöl, und Rosenhydrolat, und (iv) Lösen des Lanolins, und
(v) Abkühlen der erhaltenen Mischung.

## Claims

1. Natural substance composition consisting of peanut oil, olive oil, castor oil, and lanolin, and optionally one or more member(s) selected from the group consisting of avocado oil, rose oil, rose hydrosol, and phytosterol.

2. Composition according to claim 1, **characterized in that** it
(i) consists of peanut oil, olive oil, castor oil, and lanolin, wherein it contains peanut oil, olive oil, castor oil, and lanolin in the following concentrations:
- peanut oil 50-60 vol.%,
- olive oil 25-35 vol.%,
- castor oil 8-14 vol.%, and
- lanolin 1-4 vol.%,
wherein the selected concentrations add up to 100 vol.%; or
(ii) consists of peanut oil, olive oil, castor oil, lanolin, and rose oil, wherein it contains peanut oil, olive oil, castor oil, lanolin, and rose oil in the following concentrations:
- peanut oil 50-60 vol.%,
- olive oil 25-35 vol.%,
- castor oil 8-14 vol.%,
- lanolin 1-3 vol.%, and
- rose oil 0.001-0.020 vol.%,
wherein the selected concentrations add up to 100 vol.%; or
(iii) consists of peanut oil, olive oil, castor oil, lanolin, and rose hydrosol, wherein it contains peanut oil, olive oil, castor oil, lanolin, and rose hydrosol in the following concentrations:
- peanut oil 50-60 vol.%,
- olive oil 25-33 vol.%,
- castor oil 8-14 vol.%,
- lanolin 1-4 vol.%, and
- rose hydrosol 1-3 vol.%,
wherein the selected concentrations add up to 100 vol.%; or
(iv) consists of peanut oil, olive oil, castor oil, avocado oil, lanolin, and phytosterol, wherein it contains peanut oil, olive oil, castor oil, avocado oil, lanolin, and phytosterol in the following concentrations:
- peanut oil 50-60 vol.%,
- castor oil 25-35 vol.%,
- olive oil 5-12 vol.%,
- avocado oil 2-8 vol.%,
- lanolin 1-4 vol.%, and
- phytosterol 0.2-0.5 vol.%,
wherein the selected concentrations add up to 100 vol.%; or
(v) consists of peanut oil, olive oil, castor oil, avocado oil, lanolin, rose oil, and phytosterol, wherein it contains peanut oil, olive oil, castor oil, avocado oil, lanolin, rose oil, and phytosterol in the following concentrations:
- peanut oil 50-60 vol.%,
- castor oil 25-35 vol.%,
- olive oil 3-7 vol.%,
- avocado oil 4-8 vol.%,
- lanolin 2-4 vol.%,
- rose oil 0.001-0.020 vol.%, and
- phytosterol 0.2-0.5 vol.%,
where the selected concentrations add up to 100 vol.%.

3. Composition according to either of the preceding claims, **characterized in that** it consists of the following components:
| | |
|---|---|
| peanut oil | 55.98 vol.% |
| castor oil | 30.00 vol.% |
| olive oil | 5.00 vol.% |
| avocado oil | 5.75 vol.% |
| lanolin | 3.00 vol.% |
| rose oil | 0.02 vol.% |
| phytosterol (plant) | 0.25 vol.% |

4. Composition according to any of the preceding claims,
**characterized in that** it contains exclusively naturally occurring plant or animal substances that are unprocessed or obtained solely through mechanical or waterbased extraction methods.

5. Composition according to any of the preceding claims,
**characterized in that** the castor oil and/or the olive oil are in ozonized form.

6. Composition according to any of the preceding claims,
**characterized in that** it is in the form of an oil, a cream, a gel, a lotion, or a paste, wherein it is preferably in the form of an oil.

7. Composition according to any of the preceding claims for the topical treatment and care of psoriatic skin.

8. Composition according to claim 7, wherein the composition is applied two or three times daily to the skin to be treated.

9. Method for producing the composition according to any of claims 1 to 7, comprising the following steps:
(i) mixing peanut oil and optionally phytosterol,
(ii) heat the obtained mixture to 50 to 75°C,
(iii) adding olive oil, castor oil, and optionally one or more members selected from the group consisting of avocado oil, rose oil, and rose hydrosol, and
(iv) dissolving the lanolin, and
(v) cooling the obtained mixture.

## Revendications

1. Composition à base de substances naturelles, composée d'huile d'arachide, huile d'olive, huile de ricin et lanoline, ainsi que, éventuellement, d'un ou de plusieurs composants choisis dans le groupe composé d'huile d'avocat, huile de rose, hydrolat de rose et phytostérol.

2. Composition selon la revendication 1, **caractérisée en ce qu'**elle
(i) est composée d'huile d'arachide, huile d'olive, huile de ricin et lanoline, dans laquelle elle contient de l'huile d'arachide, de l'huile d'olive, de l'huile de ricin et de la lanoline dans les concentrations suivantes :
- 50 à 60 % en volume d'huile d'arachide,
- 25 à 35 % en volume d'huile d'olive,
- 8 à 14 % en volume d'huile de ricin, et
- 1 à 4 % en volume de lanoline,
dans laquelle les concentrations choisies sont additionnées pour donner 100 % en volume ; ou
(ii) est composée d'huile d'arachide, huile d'olive, huile de ricin, lanoline et huile de rose, dans laquelle elle contient de l'huile d'arachide, de l'huile d'olive, de l'huile de ricin, de la lanoline et de l'huile de rose dans les concentrations suivantes :
- 50 à 60 % en volume d'huile d'arachide,
- 25 à 35 % en volume d'huile d'olive,
- 8 à 14 % en volume d'huile de ricin,
- 1 à 3 % en volume de lanoline, et
- 0,001 à 0,020 % en volume d'huile de rose,
dans laquelle les concentrations choisies sont additionnées pour donner 100 % en volume ; ou
(iii) est composée d'huile d'arachide, huile d'olive, huile de ricin, lanoline et hydrolat de rose, dans laquelle elle contient de l'huile d'arachide, de l'huile d'olive, de l'huile de ricin, de la lanoline et de l'hydrolat de rose dans les concentrations suivantes :
- 50 à 60 % en volume d'huile d'arachide,
- 25 à 33 % en volume d'huile d'olive,
- 8 à 14 % en volume d'huile de ricin,
- 1 à 4 % en volume de lanoline, et
- 1 à 3 % en volume d'hydrolat de rose,
dans laquelle les concentrations choisies sont additionnées pour donner 100 % en volume ; ou
(iv) est composée d'huile d'arachide, huile d'olive, huile de ricin, huile d'avocat, lanoline et phytostérol, dans laquelle elle contient de l'huile d'arachide, de l'huile d'olive, de l'huile de ricin, de l'huile d'avocat, de la lanoline et du phytostérol dans les concentrations suivantes :
- 50 à 60 % en volume d'huile d'arachide,
- 25 à 35 % en volume d'huile de ricin,
- 5 à 12 % en volume d'huile d'olive,
- 2 à 8 % en volume d'huile d'avocat,
- 1 à 4 % en volume de lanoline, et
- 0,2 à 0,5 % en volume de phytostérol,
dans laquelle les concentrations choisies sont additionnées pour donner 100 % en volume ; ou
(v) est composée d'huile d'arachide, huile d'olive, huile de ricin, huile d'avocat, lanoline, huile de rose et phytostérol, dans laquelle elle contient de l'huile d'arachide, de l'huile d'olive, de l'huile de ricin, de l'huile d'avocat, de la lanoline, de l'huile de rose et du phytostérol dans les concentrations suivantes :
- 50 à 60 % en volume d'huile d'arachide,
- 25 à 35 % en volume d'huile de ricin,
- 3 à 7 % en volume d'huile d'olive,
- 4 à 8 % en volume d'huile d'avocat,
- 2 à 4 % en volume de lanoline,
- 0,001 à 0,020 % en volume d'huile de rose, et
- 0,2 à 0,5 % en volume de phytostérol,
dans laquelle les concentrations choisies sont additionnées pour donner 100 % en volume.

3. Composition selon l'une des revendications précédentes,
**caractérisée en ce qu'**elle est composée des constituants suivants :
| | |
|---|---|
| huile d'arachide | 55,98 % en volume |
| huile de ricin | 30,00 % en volume |
| huile d'olive | 5,00 % en volume |
| huile d'avocat | 5,75 % en volume |
| lanoline | 3,00 % en volume |
| huile de rose | 0,02 % en volume |
| phytostérol (d'origine végétale) | 0,25 % en volume |

4. Composition selon l'une des revendications précédentes,
**caractérisée en ce qu'**elle contient exclusivement des substances naturelles d'origine végétale ou animale qui ne sont pas transformées ou qui sont obtenues uniquement par des méthodes d'extraction mécaniques ou à base d'eau.

5. Composition selon l'une des revendications précédentes,
**caractérisée en ce que** l'huile de ricin et/ou l'huile d'olive se présentent sous forme ozonisée.

6. Composition selon l'une des revendications précédentes,
**caractérisée en ce qu'**elle se présente sous la forme d'une huile, d'une crème, d'un gel, d'une lotion ou d'une pâte, de préférence sous la forme d'une huile.

7. Composition selon l'une des revendications précédentes, destinée au traitement et aux soins topiques de la peau atteinte de psoriasis.

8. Composition selon la revendication 7, dans laquelle la composition est appliquée deux ou trois fois par jour sur la peau à traiter.

9. Procédé pour la préparation de la composition conformément à l'une des revendications 1 à 7, comprenant les étapes suivantes :
(i) mélange d'huile d'arachide et, éventuellement, de phytostérol,
(ii) chauffage du mélange obtenu à une température allant de 50 à 75 °C,
(iii) ajout d'huile d'olive, d'huile de ricin et, éventuellement, d'un ou de plusieurs composants choisis dans le groupe composé d'huile d'avocat, huile de rose et hydrolat de rose, et (iv) dissolution de la lanoline, et
(v) refroidissement du mélange obtenu.
